# EUROPEAN PATENT APPLICATION

(11) **EP 0 818 534 A1**
(43) Date of publication of application: **14.01.1998**
(21) Application number: 96201977.4
(22) Date of filing: 12.07.1996
(51) Int. Cl.: C12N 15/24, C12N 15/85, C12N 5/10, C12Q 1/68, A61K 35/00, A01K 67/027

(54) **Human Interleukin 12 gene expression regulating sequences**

(71) Applicant: JANSSEN PHARMACEUTICA N.V., 2340 Beerse (BE)
(72) Inventor: Van Dijck, Patrick Jozef Gerardus, 2340 Beerse (BE); Roevens, Peter Walter Maria, 2340 Beerse (BE); De Chaffoy de Courcelles, Didier Robert Guy G., 2340 Beerse (BE)
(74) Representative: Van Reet, Joseph

(57) **Abstract**

The present invention relates to human interleukin 12 (IL12) gene expression regulating sequences, including the IL-12p35 subunit gene promoter sequence, and the first intron sequence of the human IL-12p40 subunit gene, preferably operably linked to the first exon of the human IL-12p40 subunit gene and further upstream to the human IL-12p40 subunit gene promoter sequence. Further, the invention relates more particularly to DNA constructs containing such a sequence and a reporter gene, to vectors and host cells containing such a DNA construct, to a method of screening for compounds which affect the production of IL-12 in such cells, in particular human cells, to non-proteinaceous compounds obtainable by such a method, and to transgenic non-human mammalian animals which express a reporter gene under the control of one of said IL-12 gene expression regulating sequences.

## Description

The present invention relates to human interleukin 12 (IL-12) gene expression regulating sequences, more particularly to DNA constructs containing such a sequence and a reporter gene. In particular, the invention relates to host cells containing such a DNA construct and to a method of screening for compounds which affect the production of IL-12 in such cells, in particular human cells.

IL-12 is a proinflammatory cytokine composed of 35- and 40-kDa subunits and produced in particular by phagocytic cells, B cells and other types of antigen - presenting cells (APC). IL-12 mediates several biological activities on T and NK (natural killer) cells, including induction of IFN-g production, enhancement of cell-mediated cytotoxicity, and mitogenic effects. Through these functions IL-12 plays a major role in the early inflammatory response to infections. In addition, APC-produced IL-12 is critically involved in the generation of Th1 cells and is required for optimal differentiation of CTL (cytotoxic lymphocytes). The early decision toward Th1 or Th2 cells in the immune response is dependent on the balance between IL-12, which favours Th1 responses, and IL-4, which favours Th2 responses.

A lot of research has been dedicated to determine the influence which an IL-12 treatment may have on different diseases including infectious diseases (e.g. leishmaniasis, toxoplasmosis, cryptococcosis, malaria, tuberculosis, schistosomiasis), viral infections including HIV, immuno deficiencies, cancers (e.g. sarcoma, lymphoma), autoimmune diseases, diabetes,etc. The considered treatments include both administration of recombinant IL-12 protein and IL-12 gene therapy, and consistently suggest the potential usefulness of IL-12 in therapeutic applications. In addition to the above-mentioned indications relating to the immunological role of IL-12, recent findings suggest that IL-12 has anti-angiogenic effects and also haematopoietic stimulating activities. The latter may mean that IL-12 has potential in organ transplantation.

The present invention, however, does not relate to the therapeutic applications of IL-12 itself but to the genetic control of the endogenous production of IL-12 and to methods of screening for compounds which may affect said genetic control.

The genetic control of the production of IL-12 is complex because of the requirement for expression of two different genes to produce the biologically active heterodimer, more particularly the p35 and p40 subunit genes.

The cDNA's of the p35 and the p40 monomers are already known from the database GENBANK under reference p40: M65290 and p35: M65291. Recently, a promoter sequence of the IL-12 p40 subunit gene has further been identified (see Xiaojing Ma et al. (1996) J. Exp. Med., Vol. 183, 147-157). The first intron sequence of the IL-12 p40 subunit gene, which may also have an influence on the expression of this gene, and the promoter sequence of the IL-12 p35 subunit gene remained however unknown up till now.

In view of the important need for finding effective medicaments for the treatment of the above mentioned diseases, in particular cancer and AIDS, the main object of the present invention is to provide a powerful method of screening for compounds which affect the production of IL-12 in cells, especially human cells, and which may thus be useful in therapeutic treatments of these and possibly other diseases.

A first aspect of the invention provides a DNA fragment comprising at least a functional part of a human IL-12 p35 subunit gene promoter sequence or of a functional genetic variant thereof.

The expression "functional genetic variant" is intended to include DNA sequences which exert the same functions as the native promoter sequence, and also DNA sequences which comprise modifications in non-expression regulating regions, domains, in particular substitutions insertions or deletions of base pairs which do not interact with transacting factors. Especially substitutions may be considered since in this way the distance between expression regulating regions, domains is maintained.

The expression "at least a functional part" is intended to include fragments of the considered sequence which have the same or at least the most important expression regulating functions of this sequence. In case of a promoter sequence, the intended functional part thereof will usually be the part situated proximal to the first exon, upstream therefrom. On the other hand, the DNA fragments according to the present invention may comprise a longer sequence than the sequences disclosed herein.

In a particular embodiment, the IL-12 p35 subunit gene promoter sequence corresponds substantially to the SEQ ID NO:1.

The expression "corresponds substantially to" is used to indicate that the promoter sequence ID NO: 1 may possibly not be completely identical to the native human IL-12 p35 subunit gene promoter sequences. First of all because this sequence has been identified by nested PCR and secondly, because the human promoter sequence may differ somewhat from one person to another.

A second aspect of the invention provides a DNA fragment comprising at least a functional part of a first intron sequence of the human IL-12 p40 subunit gene or of a functional genetic variant thereof, which intron sequence comprises a DNA sequence corresponding substantially to the SEQ ID NO:3 proximal to the startcodon in the second exon of the human IL-12 p40 subunit gene.

In addition to the IL-12 p40 subunit gene promoter sequence, this first intron sequence may also interact with expression regulating factors, not only in the transcriptional phase but also during the post-transcriptional phase wherein it interacts in particular with splicing enzymes when being spliced from primary RNA to mRNA.

In a preferred embodiment the DNA fragment further comprises a 5' flanking region of said intron sequence including the first exon of the human IL-12 p40 subunit gene or a functional genetic variant thereof and upstream thereof and operably linked thereto at least a functional part of a human IL-12 p40 subunit gene promoter sequence or of a functional genetic variant thereof.

By combining the p40 subunit gene promoter sequence and the first exon and the first intron sequence of the p40 subunit gene, the entire expression regulating sequence is contained in the DNA fragment.

In a particular embodiment said promoter sequence corresponds substantially to the SEQ ID NO:2.

As mentioned already hereinabove, the identified sequences may possibly not be identical to the corresponding native sequences. The invention provides however further, in a third aspect, a process for the production of DNA fragments according to the invention, starting from the human genome. This process comprises screening a human genomic library using at least a portion of the SEQ ID NO:1 or of the SEQ ID NO:3 as a genetic probe.

A fourth aspect of the invention provides a DNA construct containing a DNA fragment according to the first or second aspect of the invention followed downstream by a reporter gene expressibly linked to said DNA fragment. Reporter genes code for proteins which can be detected directly by spectrophotometric, luminescent and radio-active methods, or indirectly by assays involving specific antibodies (polyclonal, but preferably monoclonal).

The reporter gene is preferably selected from the group consisting of chloramphenicol acetyltransferase (CAT), luciferase, in particular firefly luciferase and luciferases from other insects (e.g. tropical click beetle luciferases), β-galactosidase, β-glucuronidase, alkaline phosphatase, green fluorescent protein.

Different methods exist for making such DNA constructs.

A preferred method is provided in a fifth aspect of the invention, wherein the DNA construct is incorporated in a suitable vector.

Use can be made therefor of a vector already containing a reporter gene and upstream of this reporter gene a suitable locus for incorporating the DNA fragment. A variety of methods have been developed to operably link DNA fragments to such vectors via complementary cohesive termini.

A first method consists for example in generating first of all blunt-ended DNA segments by means of for example bacteriophage T4 DNA polymerase or E.coli DNA polymerase I. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to modify the DNA fragments of the invention is to use the polymerase chain reaction (PCR) as disclosed by Saiki et al (1988) Science 239, 487-491 incorporated herein by reference.

In this method, the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art. Preferably these restriction endonuclease recognition sites are different on both sides of the DNA fragment so that the DNA fragment can always be incorporated in the required direction in the expression vector. This method is certainly to be preferred in case the DNA fragments are not selected from the genome by means of a probe but by the PCR-method which has been used as described hereinafter for the identification of the DNA fragments.

A sixth aspect of the invention provides a host cell transformed with a DNA construct according to the fourth aspect of the invention.

Appropriate host cells are for example bacterium, yeast, mammalian or insect cells. These cells, in particular the bacterium cells, can be used for cloning the DNA constructs. More importantly, however, the yeast, mammal and insect cells, and in particular human cells, can be used for screening for compounds which affect the production of IL-12 in cells, especially human cells, as described hereinafter.

Transformation, including transfection, of these host cells with a DNA construct of the present invention can be accomplished by well known methods that typically depend on the type of vector used and the nature of the host cell. With regard to transformation of bacterium cells, see, for example, Cohen et al (1972) Proc. Natl. Acad. Sci. USA 69, 2110 and Sambrook et al (1989) Molecular Cloning, A Laboratory Manual, Cold Spring Harbor Laboratory, Cold Spring Harbor, NY. Transformation of yeast cells is described in Sherman et al (1986) Methods in Yeast Genetics, A Laboratory Manual, Cold Spring Harbor, NY. The method of Beggs (1978) Nature 275, 104-109 is also useful. Mammal and insect cells can be transfected by using reagents such as for example calcium phosphates and DEAE - dextran or liposome formulations.

Electroporation is also useful for transforming cells and is well known in the art for transforming yeast cells, bacterial cells and animal or human cells. For example, many bacterial species may be transformed by the methods described in Luchansky et al (1988) Mo. Microbiol. 2, 637-646 incorporated herein by reference. The greatest number of transformants is consistently recovered following electroporation of the DNA-cell mixture suspended in 2.5X PEB using 6250V per cm at 25mFD.

Methods for transformation of yeast by electroporation are disclosed in Becker & Guarante (1990) Methods Enzymol. 194, 182.

Successfully transformed cells, eg cells that contain a DNA construct of the present invention, can be identified by well known techniques. For example, cells resulting from the introduction of an expression construct of the present invention can be harvested and lysed and their DNA content examined for the presence of the DNA using a method such as that described by Southern (1975) J. Mol. Biol. 98, 503 or Berent et al (1985) Biotech. 3, 208 or by the polymerase chain reaction.

The DNA constructs according to the present invention can further also be introduced by microinjection or by using retroviruses into certain host cells, in particular in embryo cells as described hereinafter for making transgenic animals.

The DNA construct incorporated in the host cell may contain a DNA fragment according to the first aspect of the invention corresponding to the IL-12 p35 subunit gene promoter sequence.

The DNA construct incorporated in the host cell may alternatively contain a DNA fragment according to the second aspect of the invention corresponding to the first intron sequence of the IL-12 p40 subunit gene and including preferably also a sequence corresponding to the IL-12 p40 subunit gene promoter sequence so as to have the entire IL-12 p40 subunit production regulating sequence. Such an entire sequence is very advantageous in view of selecting compounds, as described hereinafter, which may have an influence on the expressional regulation, either on the transcriptional and/or on the post-transcriptional level, of the IL-12 p40 subunit production. In other words, without the first intron sequence of the IL-12 p40 subunit gene, compounds which have a direct or indirect influence on expression regulating factors which might cooperate only with this intron sequence could otherwise not be selected.

In a preferred embodiment, the host cell is transformed both with a DNA construct containing a DNA fragment according to the first aspect of the invention and with DNA construct containing a DNA fragment according to the second aspect of the invention, or with a DNA construct containing a functional part of a human IL-12 p40 submit gene promoter sequence or of a functional genetic variant thereof.

Such a double transformed host cell is particularly suited for selecting by only one test method compounds which influence the p35 subunit production or the p40 subunit production. In such test, the two different DNA constructs may contain the same or a different reporter gene.

Preferably, the mammalian, yeast or insect cells used in such test method are stably transfected with the DNA construct or constructs. Methods for making such stable cell lines are generally known and are described for example in R.W. Old et al. Principles of Gene Manipulation, 5th Ed., Blackwell Science, 1994. Use can for example be made of DNA constructs containing in addition to the above described reporter gene a positive selection gene, in particular an anti-toxicity gene which enables the stably transfected cells to survive in the presence of the corresponding toxic challenge, for example neomycine, ampicillin, G418 (genetricin), ouabain and the like.

Host cells which are especially preferred for use in the screening methods described hereinafter include cells which produce in vivo IL-12, e.g. monocytes, macrophages, neutrophils, B cells optionally transformed with an Epstein-Barr virus, and also cells which do not produce IL-12 or only a too small amount, such as HIV infected cells, including T cells.

In addition to the transformed host cells, the present invention contemplates of course also a culture of those cells, preferably a monoclonal culture, or a culture derived from a monoclonal culture in a nutrient medium, tissues, tissue cultures and even organs.

A seventh aspect of the invention provides a method for screening for compounds which affect the production of IL-12 in cells, especially human or other animal cells.

This method comprises the steps of :
- contacting a compound with a cell according to the sixth aspect of the invention ; and
- testing the influence of the presence of said compound on the expression of the reporter gene.

Use can be made of yeast, mammalian or insect cells but the use of human cells is most preferred since the expression regulating mechanisms of the other cells may be different from the expression regulating mechanisms of human cells.

When screening for compounds which affect the production of IL-12 in particular in human cells, use is preferably made of IL-12 producing cells such as monocytes, macrophages, neutrophils, B cells optionally transformed with an Epstein-Barr virus, etc, the use of monocytes being in particular preferred. In this way, both compounds which have a positive or a negative effect on the IL-12 production can be identified.

When screening for compounds which lower the IL-12 production, the cells can be contacted with these compounds in the presence of an added inducer and/or enhancer of the IL-12 production, in particular in the presence of added IFN-g, GM-CSF, lipopolysaccharide (LPS), endotoxins and the like.

On the other hand, it is known that HIV virus infections cause a reduced IL-12 production so that it is believed that compounds which bring about an increased IL-12 level could be effective in treating such infections. Such compounds can in particular be selected by making use of HIV infected human cells, in particular T cells, transfected with one or more DNA sequences according to the present invention.

Further, it is also possible according to the present invention to screen for compounds which increase the IL-12 production by contacting the cells with these compounds in the presence of an added repressor and/or silencer of the IL-12 production, in particular in the presence of added IL-10, IL-4, IL-13, TGF-β, .... Such tests may further also help in determining the way wherein the selected compounds exert their influence on the IL-12 production. This is also the case for the test described hereinabove involving the use of inducers and/or enhancers.

An eight aspect of the invention relates to non-proteinaceous compounds, different from lipopolysaccharide, and obtainable by the method according to the seventh aspect of the invention for use as a medicament in a therapeutic treatment, whilst a ninth aspect of the invention relates to the use of these compounds in the manufacture of a medicament for the treatment a disease involving a too high or too low IL-12 production, in particular a disease from the group of cancers, viral infections including HIV, immuno deficiencies, autoimmune diseases, infectious diseases, or allergic diseases.

In this eight and ninth aspect of the invention, the use of lipopolysaccharide, which stimulates the IL-12 production in vitro, is excluded i.a. because lipopolysaccharide is not suited for therapeutic applications.

A tenth aspect of the invention relates to a method of making a transgenic non-human mammalian animal which expresses a reporter gene under the control of a DNA fragment according to the first and/or second aspect of the invention, i.e. under the control of a human IL-12 p35 subunit gene promoter sequence and/or under the control of a human IL-12 p40 subunit gene expression regulating sequence.

This method comprises the steps of (a) introducing into a embryo cell of said animal a DNA construct according to the fourth aspect of the invention, (b) introducing the thus obtained recombinant embryo into a pseudopregnant female animal, (c) sustaining the female animal until the embryo has developed sufficiently to be independent of its mother ; and (d) sustaining said independent transgenic animal.

The expression "transgenic" animal is intended to include animals having a genetic construction different from the normal animal of that species. In this case, the DNA of the transgenic animal comprises a human IL-12 subunit gene expression regulating sequence followed downstream by a reporter gene expressibly linked to this expression regulating sequence. The DNA of the transgenic animal comprises more particularly a DNA construct according to the fourth aspect of the invention. Such a DNA construct differs from the native DNA of the animal species especially by the fact that it contains a non-native combination of a reporter gene and an IL-12 subunit gene expression regulating sequence.

In this tenth and also in the eleventh aspect of the invention, described hereinafter, preferred non-human mammalian animals include mouse, rat, pig (including "mini-pigs" and "micro-pigs"), sheep, guinea pig and primates.

For making the transgenic animal, use is made of DNA constructs of the fourth aspect of the invention, or of vectors according to the fifth aspect of the invention. These DNA constructs or vectors are introduced into the embryo cells and the embryo cells are grown conveniently in utero following implantation to produce transgenic offspring.

For introducing the DNA constructs or vectors into the embryo cells, use can be made for example of retroviruses or standard microinjection methods such as are described in Kraemer et al (1985), Costantini and Jaenisch, eds., Genetic Manipulation of the Early Mammalian Embryo, Cold Spring Harbor Laboratory (bovine embryo microinjection); Hammer et al (1985) Nature 315, 680 (rabbit, sheep, and porcine embryo microinjection); and Gordon and Ruddle (1984) Methods in Embryology 1021, 411 (mouse embryo microinjection). Germ-line transformation of mice is also described by Palmiter & Brinster (1986) Ann. Rev. Genet. 20, 465-499. Microinjection is preferably carried out on an embryo at the one cell stage, to maximize both the chances that the injected DNA will be incorporated into all cells of the animal and that the DNA will also be incorporated into the germ cells, so that the animal's offspring will be transgenic as well. Microinjection is a standard technique which involves, briefly, isolating fertilized ova, visualizing the pronucleus, and then injecting the DNA into the pronucleus by holding the ova with a blunt holding pipette of a diameter on the order of 50 mm, and using a sharply pointed pipette of a diameter on the order of 1.5 mm to inject buffer-containing DNA into the pronucleus.

Production of transgenic non-human mammals including pigs is also described in WO 94/29434; WO94/26884; WO94/10305; WO94/05796; WO94/04672; WO93/25669; WO93/25071; EP-A-O 560 156; WO 92/22646; WO 94/01040 and WO 91/05855.

All of these references describing the production of transgenic animals are incorporated herein by reference.

An eleventh aspect of the invention provides a transgenic non-human mammalian animal which expresses a reporter gene under the control of a DNA fragment according to the first aspect of the invention, and which may preferably further express a further reporter gene under the control of a human IL-12 p40 subunit gene expression regulating sequence or of a functional genetic variant thereof, and also a transgenic non-human mammalian which expresses a reporter gene under the control of a DNA fragment according to the second aspect of the invention.

Such transgenic animals are particularly suited for making studies of the effects of the compounds obtainable by the screening method of the seventh aspect of the invention, and in particular also the location of these effects in the animal body. Conveniently, the transgenic animals are made by the method of the tenth aspect of the invention or are formed by descendants of transgenic animals made by such method.

In another embodiment, the invention provides a method for screening for a nucleic acid binding protein which regulates IL-12 transcription by a cell. The method includes the steps of contacting a nucleic acid containing the sequences according to the first and/or second aspect of the invention with a mixture of proteins, and isolating a protein which specifically binds to such a sequence, an indication that the nucleic acid binding protein regulates transcription of IL-12.

Also included is a screening method to identify a candidate compound capable of affecting the level of IL-12 transcription in cells, by contacting a nucleic acid which contains a sequence according to the first and/or second aspect of the invention, in the presence and in the absence of a candidate compound, with a cellular protein which binds to the nucleic acid in vivo, and determining whether the binding of the cellular protein to the DNA is altered in the presence of a candidate compound, indicating that the candidate compound affects the level of IL-12 transcription.

The invention also includes a method of identifying a compound which affects the level of IL-12 transcription in celles, by (1) providing a DNA containing the sequence according to the first and/or the second aspect of the invention operatively linked to a sequence encoding a reporter gene product, (2) contacting the DNA with a candidate compounds in a cell or cell lysate, and (3) determining whether the level of transcription of the reporter gene is altered in the presence of the candidate compound.

In yet another embodiment, the invention includes a method of regulating transcription of a gene by introducing a nucleic acid containing a sequence according to the first and/or the second aspect of the invention to the transcriptional start of the gene, wherein the gene does not encode IL-12.

Further details of the invention will become apparent from the following examples given with reference to the annexed drawings wherein :
Figure 1 shows the organisation of the promoter region of IL12p35 ; and
Figure 2 shows the organisation of the first intron of IL12p40.

In these figures, the following abbreviations have been used :
Pv : PvuII ; Bg : BglII ; Sc : ScaI ; Dr: Dra I ; Ec: EcoRV ; Ss : SspI ; Ml : MluI.

### Example 1

### Isolation of the human IL12p35 promoter

Interleukin 12 is a cytokine that is made up of two polypeptides. The covalent binding of these two polypeptides results in a bioactive product. The cDNAs of these two subunits have been described in the literature (Wolf et al., 1991). Very recently the promoter of the p40 subunit has been described (Ma et al., 1996). To obtain the promoter of the human IL12p35 subunit gene use has been made of the promoterFinder DNA walking kit from Clontech. This kit contains five "libraries" of uncloned, adaptor-ligated human genomic DNA fragments. It allows researchers to walk upstream (i.e. towards promoters) or downstream in genomic DNA starting from a known sequence such as a cDNA. The adaptor contains two primer binding sites and a number of restriction sites.

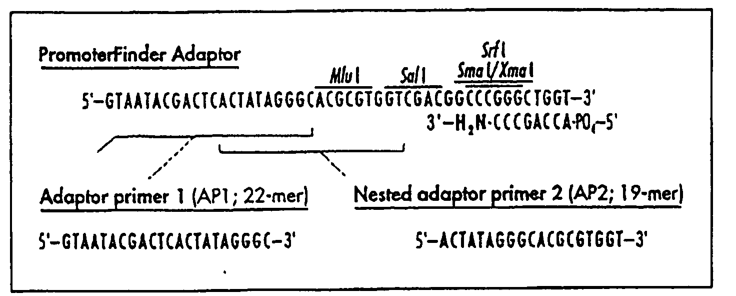
PromoterFinder Adaptor
Srf I
Mlu I Sal I Sma I/Xma I
5'-GTAATACGACTCACTATAGGGCACGCGTGGTCGACGGCCCGGGCTGGT-3'
3'-H2N-CCCGACCA-PO4-5'
Adaptor primer 1 (AP1; 22-mer) Nested adaptor primer 2 (AP2; 19-mer)
5'-GTAATACGACTCACTATAGGGC-3' 5'-ACTATAGGGCACGCGTGGT-3'

Two IL12p35 specific primers were designed based on the available cDNA sequence for the IL12p35 subunit..

1 GGTCACCGAG AAGCTGATGT AGAGAGAGAC ACAGAAGGAG ACAGAAAGCA

51 AGAGACCAGA GTCCCGGGAA AGTCCTGCCG CGCCTCGGGA CAATTATAAA

101 AATGTGGCCC CCTGGGTCAG CCTCCCAGCC ACCGCCCTCA CCTGCCGCGG

151 CCACAGGTCT GCATCCAGCG GCTCGCCCTG TGTCCCTGCA GTGCCGGCTC

201 AGCATGTGTC CAGCGCGCAG CCTCCTCCTT GTCGCTACCC TGGTCCTCCT

The gene specific primers are :
IL12p35GSP3: 5' CCCGGGACTCTGGTCTCTTGCTTTCTGTC 3'
IL12p35GSP4: 5' CGAGATCTGTCTCTCTCTACATCAGCTTCTCGGTG 3'

GSP4 comprises an artificial built-in recognition site for BglII which is underlined.

Two consecutive rounds of PCR, where the second PCR is a nested PCR, were performed.

In the first PCR use was made of the IL12p35GSP3 primer and the adaptor specific primer 1 (AP1). Each PCR reaction contained:
- 37.8 µl: water
- 5 µl: 10X Tth PCR reaction buffer
- 1 µl: dNTP (10 mM each)
- 2.2 µl: Mg(OAc)₂ (25 mM)
- 1 µl: AP1 (10 µM)
- 1 µl: IL12p35GSP3 (10 µM)
- 1 µl: Advantage Tth Polymerase mix (50 X)
- 1 µl: DNA library

The 50X polymerase mix was made by combining 20 µl Tth polymerase (2U/µl, Clontech) with 1 µl Vent polymerase (2U/µl, New England BioLabs) and 5 µl of TthStart antibody (Clontech).

The first PCR was performed under the following conditions in an MJ PTC200 machine using a heated lid:
- 7 cycles:: 94 °C 25 sec
72 °C 4min
- 32 cycles:: 94 °C 25 sec
67 °C 4 min
67 °C for an additional 4 min after the final cycle.

8 µl of the primary PCR reaction products were analyzed on a 1 % agarose/EtBr gel along with molecular weight marker VII (Boehringer Mannheim).
On the gel a light smear of PCR products could be seen.

Subsequently, the nested PCR reaction was performed. In this second PCR use was made of the IL12p35GSP4 primer and the adaptor specific primer 2 (AP2). Each reaction contained:
- 37.8 µl: water
- 5 µl: 10X Tth PCR reaction buffer
- 1 µl: dNTP (10 mM each)
- 2.2 µl: Mg(OAc)₂ (25 mM)
- 1 µl: AP2 (10 µM)
- 1 µl: IL12p35GSP4 (10 µM)
- 1 µl: Advantage Tth Polymerase mix (50 X)
- 1 µl: PCR product of first PCR (1/50 dilution)

The conditions for the second PCR were identical as for the first PCR exept that only 20 cycles were performed instead of 32.

10 µl of the secondary PCR reaction products were analyzed on a 1 % agarose/EtBr gel along with molecular weight marker VII (Boehringer Mannheim).

In one of the 5 libraries (HDL 4) there was an amplification product of ± 1300 bp.

### Example 2.

### Construction and characterization of a reporter construct with the human IL12p35 promoter.

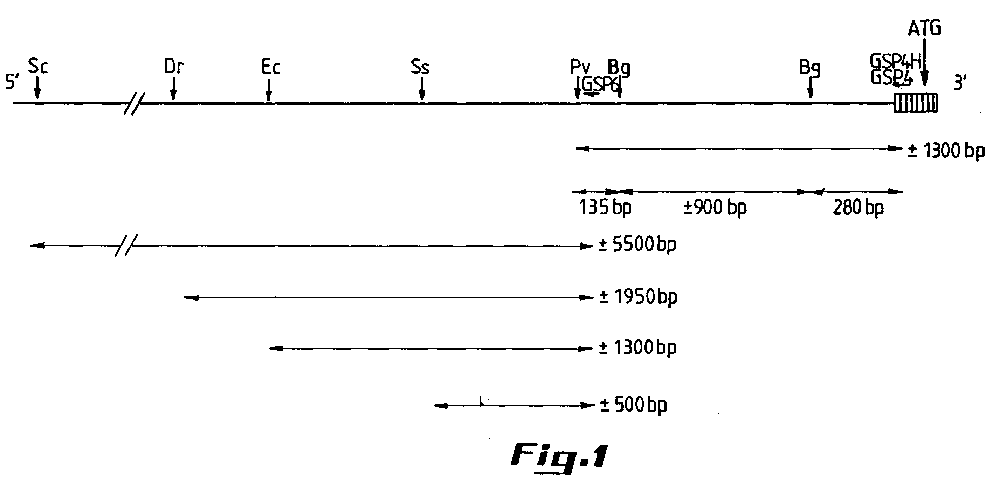

After purification of the 1300 bp fragment it was digested with MluI (present in the adaptor) and BglII (built in in primer GSP4). This digest resulted in three fragments of approximately 900, 280 and 135 bp (Figure 1). The same fragments were obtained using a single BglII digest. This means that there are two internal BglII sites. After purification of the fragments they were subcloned in pGL2basic. This is an eukaryotic expression vector containing the luciferase reporter gene (Promega corp.). The two largest fragments were subcloned in the BglII site. The 135 bp fragment was subcloned in the MluI and BglII sites.

To obtain the complete 1300 bp fragment use was made of a modified GSP4 primer. The new primer (IL12p35GSP4H) contained a HindIII recognition sequence instead of a BglII site. There was no HindIII recognition site in either of the three BglII fragments.

This primer was used together with the adaptor specific primer 2 in a nested PCR reaction. As template use was made of 1 µl of a 1/50 dilution of the PCR reaction products of the first PCR with HDL4 as template. After agarose gel electrophoresis the 1300 bp fragment was digested with MluI and HindIII and the fragment was subcloned in the pGL2basic vector and subsequently sequenced.

Based on the sequence of this clone, two new primers were designed, namely GSP5 and GSP6, which are localized in the 5' upstream region of that clone. These primers are :
IL12p35GSP5 : 5' TGACAGAGACATGTCAGAGTAGCAGCCTCTG 3'
IL12p35GSP6 : 5' TCAGATCTCTCCACCCTAAATTCAGAGAGTAGCTCTTC 3'

GSP6 comprises an artificial built-in recognition site for BglII which is underlined.

These primers were successively used again, in combination with the adapter specific primers AP1 and AP2, in the promoter finder kit. In one of the 5 libraries (HDL5) there was an amplification product of ± 500 bp, (see Figure 1, pGLIL12p35GSP6HD5). This latter fragment was also cloned in the pGL2 basic vector and subsequently sequenced.

The combined sequence of the ± 1300 bp fragment and the ± 500 bp fragment corresponds to SEQ ID NO:1.

In the other libraries of the used promoter finder kit, longer fragments could be found on the basis of the same primers GSP5 and GSP6 which, after cloning, can also be sequenced. As shown in Figure 1, these fragments had a length of ± 1300 bp (pGLIL12p35GSP6HD1), ± 1950 bp (pGLIL12p35GSP6HD3) and ± 5500 bp (pGLIL12p35GSP6HD2) and enable thus to prepare an IL12p35 promoter sequence having a length longer than SEQ ID NO:1.

### Putative cis-acting sequences present in the IL12p35 promoter

The promoter does not contain a TATA-box at the appropriate position. This is similar to the promoter of the murine IL12p35 gene (Yoshimoto et al., 1996). The promoter contains binding sites for some general as well as for some specific transcription factors. Among the general transcription factors there are binding sites for AP-2, SP-1, C/EBP and NF-E1. Binding sites for more specific transcription factors include a Pu-box and a Ets-1 consensus sequence. Ets family transcription factors have been shown to be important for transcriptional regulation of other cytokines (PU-1 for IL 1β (Kominato Y. et al., 1995) and ets-1 for TNFa (Krämer B et al., 1995)).

### Determination of the transcription initiation site

The transcription initiation site was determined using the method of primer extension. The RNA was prepared from either NC37 or THP1 cells. The primer used was IL12p35GSP3.

Briefly, 15 µg of total RNA was hybridized with 0.7 ng primer (1X10⁸ cpm/µg) for 20 min at 58 °C in 10 µl of 50 mM Tris-Cl (pH 8.3 at 42 °C), 50 mM KCl, 10 mM MgCl2, 10 mM DTT, 1 mM dNTP, 2 U Rnase inhibitor and 0.5 mM spermidine. After cooling down to room temperature 10 µl (1 U) of .M-MuLV.reverse transcriptase was added and the mixture was incubated for 30' at 42 °C.

The primer extension products were purified by Phenol/chloroform/isoamylalcohol (25:24:1) extraction and precipitation with EtOH. After drying, the products were resuspended in loading buffer and analyzed on a 6 % acrylamide sequencing gel.

Instead of one predominant band there were 4 signals. This is typical for promoters that lack a TATA box. 4 transcription initiation sites have also been reported in the mouse promoter (Yoshimoto et al., 1996). The 4 alternative start sites were found in the human promoter are underlined in SEQ ID NO:1, respectively in positions 1352, 1594, 1625 and 1626. This means that SEG ID NO:1 contains a part of the first exon of the IL12p35 subunit gene. This part may thus possibly be eliminated from the fragment according to the first aspect of the invention, either partially or entirely.

### Example 3

The expression vector was analyzed by transient transfection into THP1 cells (ATCC TIB 202). THP1 cells are human premonocytic cells. Positive (pGL2control) and negative (pGL2basic) control vectors were included in the assay.

THP1 cells were grown in RPMI-1640 to which the following components were added: 10 % heat inactivated foetal bovine serum, 5 ml βmercapto ethanol stock, 6 ml glutamine and 2.5 ml penicilline/streptomycin.

The βmercapto ethanol stock was made as follows: 20 ml RPMI-1640 + 10% heat inactivated FBS + 7 µl βmercapto ethanol.

### Example 4

### Isolation of the first intron of IL12p40

### a: cDNA sequence

Accession number: M65272

1 CTGTTTCAGG GCCATTGGAC TCTCCGTCCT GCCCAGAGCA AGATGTGTCA

51 CCAGCAGTTG GTCATCTCTT GGTTTTCCCT GGTTTTTCTG GCATCTCCCC

101 TCGTGGCCAT ATGGGAACTG AAGAAAGATG TTTATGTCGT AGAATTGGAT

151 TGGTATCCGG ATGCCCCTGG AGAAATGGTG GTCCTCACCT GTGACACCCC

### b: Gene specific primers:

IL12p40PR2: 5' CGAGGGGAGATGCCAGAAAAACCAGGGAAAACC 3'
IL12p40GSP3: 5' GTAGATCTCAAGAGATGACCAACTGCTGGTGACAC 3'
The last C corresponds to the G of the ATG startcodon. An artificial recognition site for BglII is underlined.

### c: Results

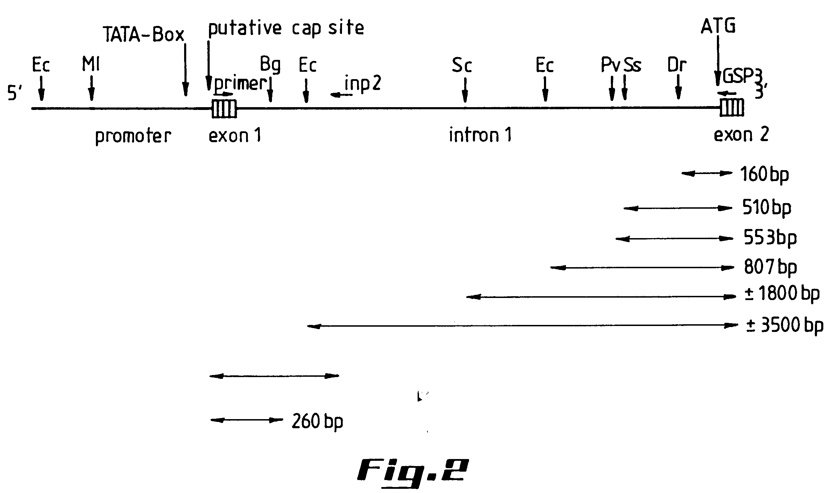

The products of the PCR reaction were separated on an agarose/EtBr gel (see Fig. 2)

In each lane (=different library) there is a clear distinct fragment. Furthermore it seems that in lane 1 (corresponding to library 1) there are two fragments. This can occur if the original genomic DNA was not completely digested with, in this case, EcoRV.

After isolation from the gel the fragments were digested with MluI (present in the adaptor) and BglII (present in GSP3) and subcloned in pGL2basic upstream of the luciferase reporter gene.

The sequence of all these clones has been determined and can be found back in SEQ ID NO:3. From the sequence it appeared that there is an intron upstream of the ATG startcodon:
1. The sequence upstream of the startcodon does not fit with the sequence of the published 5' untranslated region.
2. Just in front of the startcodon there is an AG dinucleotide which are always the two nucleotides at the 3' border of an intron
3. 5' from this AG sequence there is a 15 bp pyrimidine rich region which is typical for an intron.
4. The publication by Ma et al., 1996, demonstrates the presence of an intron just in front of the start codon.

Figure 2 shows an overview of all the fragments and the entire sequence corresponds to the SEQ ID NO:3.

### Example 5

### Isolation of the promoter of IL12p40

### a: cDNA sequence

Accession number: M65272

1 CTGTTTCAGG GCCATTGGAC TCTCCGTCCT GCCCAGAGCA AGATGTGTCA

51 CCAGCAGTTG GTCATCTCTT GGTTTTCCCT GGTTTTTCTG GCATCTCCCC

101 TCGTGGCCAT ATGGGAACTG AAGAAAGATG TTTATGTCGT AGAATTGGAT

151 TGGTATCCGG ATGCCCCTGG AGAAATGGTG GTCCTCACCT GTGACACCCC

### b: Gene Specific primers:

IL12P40GSP4: 5' TTGCTCTGGGCAGGACGGAGAGTCC 3'
IL12GSP2: 5' GTAGATCTGACGGAGAGTCCAATGGCCCTGAAAC 3'
An artificial recognition site for BglII is underlined.

### c: Results

Only in library 1 there was a fragment of approximately 950 bp. After purification the fragment was digested with MluI and BglII. This digest resulted in two fragments of approximately 650 and 300 bp. The 650 bp fragment was subcloned in pGL2basic digested with MluI and BglII. The 300 bp fragment was subcloned in the same vector digested with MluI alone.

The sequence of both fragments has been determined and is presented as SEQ ID NO:2. There is evidence that this fragment contains the promoter region of IL12p40.
1. The sequence is located 5' upstream of the cDNA
2. Approximately 35 bp in front of the putative transcription start site there is a perfect TATA box sequence.
3. The fragment contains binding sites for transcription factors that are enriched or even specific for B cells and macrophages. One such factor is PU.1.
4. Apart from 3 mismatches our sequence is identical to the sequence in the paper by Ma et al., 1996.

### REFERENCES

Wolf SF., Temple, PA., Kobayashi M., Young D., Dicig, M., Lowe L., Dzialo, R., Fitz L., Ferenz C., Hewick R.M., Kellehe K., Herrmann SH., Clarc SC., Azonni L., Chan SH. Trinchieri G. and Perussia B. Cloning of cDNA for natural killer cell stimulatory factor, a heterodimeric cytokine with multiple biologic effects on T and natural killer cells. J. immunol. 146, 3074-3081, 1991.
Ma X., Chow JM., Gri G., Carra G., Gerosa F., Wolf SF., Dzialo R. and Trinchieri G. The interleukin 12 p40 gene promoter is primed by interferon gamma in monocytic cells. J. exp. Med., 183, 147-157, 1996.
YoshimotoT., Kojima K., Funakoshi T., Endo Y., Fujita T. and Nariuchi H. Molecular cloning and characterization of murine IL-12 genes. J. Immunol. 156, 1082-1088, 1996.
Krämer B., Wiegmann K. and Krönke M. Regulation of the human TNF promoter by the transcription factor Ets. J. Biol. Chem., 270, 6577-6583, 1995.
Kominato Y., Galson DL., Waterman WR., Webb AC. and Auron PE. Monocyte expression of the human prointerleukin 1β gene (IL1B) is dependent on promoter sequences which bind the hematopoeitic transcription factor Spi-1/PU.1. Mol. Cell. Biol. 15, 58-68, 1995.

## Claims

1. A DNA fragment comprising at least a functional part of a human IL-12 p35 subunit gene promoter sequence or of a functional genetic variant thereof.

2. A DNA fragment according to claim 1, wherein said promoter sequence corresponds substantially to the SEQ ID NO:1.

3. A DNA fragment comprising at least a functional part of a first intron sequence of the human IL-12 p40 subunit gene or of a functional genetic variant thereof, which intron sequence comprises a DNA sequence corresponding substantially to the SEQ ID NO:3 proximal to the startcodon in the second exon of the human IL-12 p40 subunit gene.

4. A DNA fragment according to claim 3, further comprising a 5' flanking region of said intron sequence including the first exon of the human IL-12 p40 subunit gene or a functional genetic variant thereof and upstream thereof and operably linked thereto at least a functional part of a human IL-12 p40 subunit gene promoter sequence or of a functional genetic variant thereof.

5. A DNA fragment according to claim 4 wherein said promoter sequence corresponds substantially to the SEQ ID NO:2.

6. A process for the production of a DNA fragment as set out in any one of the claims 1 to 3 which process comprises screening a human genomic library using at least a portion of the SEQ ID NO:1 or of the SEQ ID NO:3 as a genetic probe.

7. A DNA construct containing a DNA fragment according to any one of the claims 1 to 5 followed downstream by a reporter gene expressibly linked to said DNA fragment.

8. A DNA construct according to claim 7 wherein the reporter gene is selected from the group consisting of luciferase, chloramphenicol acetyltransferase, β-glucuronidase, alkaline phosphatase and green fluorescent protein.

9. A vector containing a DNA construct according to claim 7 or 8.

10. A host cell transformed with a DNA construct according to claim 7 or 8.

11. A host cell according to claim 10 wherein the DNA construct contains a DNA fragment according to claim 4 or 5.

12. A host cell according to claim 10 or 11 wherein the DNA construct contains a DNA fragment according to claim 1 or 2.

13. A host cell according to claim 12 wherein the cell is further transformed with a further DNA construct containing at least a functional part of a human IL-12 p40 subunit gene promoter sequence or of a functional genetic variant thereof, followed downstream by a reporter gene expressibly linked to this latter promoter sequence.

14. A host cell according to any one of the claims 10 to 13 wherein the cell is any of a bacterium, yeast, mammalian or insect cell.

15. A host cell according to any one of the claims 10 to 14, wherein the cell is a mammalian, yeast or insect cell stably transfected with said DNA construct and/or with said further DNA construct.

16. A host cell according to any one of the claims 10 to 15 wherein the cell is selected from the group consisting of monocytes, macrophages, neutrophils, B cells optionally transformed with an Epstein-Barr virus, HIV infected cells, or T cells.

17. A cell derived from a host cell according to any one of the claims 10 to 16.

18. A method of screening for compounds which affect the production of IL-12 in cells, especially human cells, comprising the steps of :
- contacting a compound with a cell according to any one of the claims 10 to 17, preferably with a cell according to claim 16 and/or with cells derived therefrom ; and
- testing the influence of the presence of said compound on the expression of said reporter gene.

19. A method according to claim 18 wherein said cell is contacted with said compound in the presence of an added inducer and/or enhancer of the IL-12 production, in particular in the presence of added IFN-g, GM-CSF, lipopolysaccharide, antitoxins and the like.

20. A method according to claim 18 wherein said cell is a monocyte.

21. A method according to claim 18 wherein said cell is contacted with said compound in the presence of an added repressor and/or silencer of the IL-12 production, in particular in the presence of added IL-10, IL-4, IL-13, TGF-β.

22. A non-proteinaceous compound different from lipopolysaccharide and obtainable by the method according to any of the claims 18 to 21 for use as a medicament in a therapeutic treatment.

23. Use of a non-proteinaceous compound different from lipopolysaccharide and obtainable by the method according to any one of the claims 18 to 21 in the manufacture of a medicament for the treatment of a disease involving a too high or too low IL-12 production, in particular a disease from the group of cancers, viral infections including HIV, immuno deficiencies, autoimmune diseases, infectious diseases, or allergic diseases.

24. A method of making a transgenic non-human mammalian animal which expresses a reporter gene under the control of a DNA fragment according to any one of the claims 1 to 5 comprising the steps of :
(a) introducing into an embryo cell of said animal a DNA construct according to claim 7 or 8 ;
(b) introducing the thus obtained recombinant embryo into a pseudopregnant female animal ;
(c) sustaining the female animal until the embryo has developed sufficiently to be independent of its mother ; and
(d) sustaining said independent transgenic animal.

25. A transgenic non-human mammalian animal which expresses a reporter gene under the control of a DNA fragment according to claim 1 or 2 to which said reporter gene is expressibly linked.

26. A transgenic animal according to claim 25 which expresses a further reporter gene under the control of a human IL-12 p40 subunit gene expression regulating sequence or of a functional genetic variant thereof to which said further reporter gene is express-ibly linked.

27. A transgenic non-human mammalian animal which expresses a reporter gene under the control of a DNA fragment according to claim 4 or 5 to which said reporter gene is expressibly linked.


SEQUENCE LISTING

(1) GENERAL INFORMATION:

(i) APPLICANT:
(A) NAME: Janssen Pharmaceutica N.V.
(B) STREET: Turnhoutseweg 30
(C) CITY: Beerse
(E) COUNTRY: Belgium
(F) POSTAL CODE (ZIP): 2340

(ii) TITLE OF INVENTION: Human Interleukin 12 gene expression
regulating sequences

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 1679 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO: 1:

ATTTGAGTGT CTCCGATGGC CTTGCTAGGA CCAACAGATC ATTTACTTGG AAGAGCTGGT 60

TCCTAGAATT TGGGTTAGTC AGAGCTGCAT CTTAGACAGT CCCCGTGACC TGTCATTAGC 120

CCCATGGGAG ACGGGCATCT CTCCACCTAA TGGTGGGCTG GTAGTGACAG CCCCAGAGGG 180

TTTCATGAGA GGAGGGCACC AGCATTGCTC GGGTGGAGAA TCTCACAAGA TCCCTCAGGC 240

AGATAAGCCT TTTGGATCAG TGGCCTGTCA GCTACCCGAG AATGTTCTGA AGGCTCTGAT 300

TTGCCTCAAA GCTGGAAAAA ACTCACTAGG GTCATTTTCC TGTTCAAAGA TATAAAGGGC 360

AAGCCTCGCT TCCCTTGGAT TCCCTTAATG TTCTATTTGG TTGGTGCAAA AGTAATTACT 420

TTTGTACCAA CCTAATACCA TGCCTCTTGC AGCTGCTCCT TGAAGAGCTA CTCTCTGAAT 480

TTAGGGTGGA GAAACTGGTG CCCCGAGCAG AGGCTGCTAC TCTGACATGT CTCTGTCAAC 540

CGGGAGGGCC GCATGACATG GATCTATATC CTAAAAGATT CAGATCTATA GTANAAAAGG 600

AGTCTTGCTT TGTTGTCTAG GCTGATCTCA AACTCCTGGC CTCAAGGTAA TCCTCCTGCC 660

TCAGCCTCCC AAAGTATTTA ACCCTTTTAT TGCAAAATAA AACACACTTA TAGAAAGCCA 720

CACAAAATAA ACATATAACT TAATGGATTA TTATAAGATG CACTGTATTA TAGTCAACTC 780

TGGGGCAATA AATAGATCCC TCTGGTGTCC CATCTCAATC TCAATGCTTC CTCACTCCCT 840

GAAGGTGGAC ACTCTCCTGA CTTACAGAAT TGTCCCTTCT TTGCATCTCC TTATAGCTTT 900

GGCACCAGGT GGGCATTTCT GGATGCACAG TGTAGCCTTG ACCCTTTTAA AACACGGGAT 960

GTTGTTTTTT TTTATCTCTT TTTATCTGCC TTTTTTGTTT CCTTTACAAC TTTTTGTTTC 1020

CTTACAACTT CTATTGAGGG ATCTGGGACA TTTGGGCTGA GGATCTGAAA CTGGGCAATT 1080

GAACAAAAAG ACAGACAAAT AAATGAACAG GCTGGGTTTC CACTTTGACT TCCCTCAGTT 1140

TATTTTCCAG GAGCTACTGG GGCAGGGGAG AGGAAAATTA TGTGAATGGA GTGGAGTAGG 1200

TATTAGGAGA GTCAACGGGG CATTTGATAA GCATGTGACC GTGGTTTCCA GGCTCACAGG 1260

TGTGGCTGAG GGTCACCAGC CCGGCTCCCC GCCCCCGACA GTCCCTGAGC TTGGGCTTTA 1320

GCTTGGAGTG GTGGATGGGA AAGCTGCTAT AGTCTCCCGG AGTGAAAAGC CCAAAGGCTT 1380

TATCCCTGTC AAGAGCAGAT CTGACAAGAC AAAGGCAGCC TGGTAACCAG AGAGCCAGTG 1440

GGGAGTTGGA ACACCTGCCT GCCCTTCCGG CTTCTGAAAG CCTCCCTTCC CCACTCTGGG 1500

CCTCAGTTTC TCCCCTTGGC AACGTGCAGA TGTGAGCATC CAACTTATGG AAGCATGCGG 1560

TCACGCATGT TTGCAGATCC CTGGCACCCC TGGGGAGTTA ATCCGAAAGC GCCGCAAGCC 1620

CCGCGGGCCG GCCGCACCGC ACGTGTCACC GAGAAGCTGA TGTAGAGAGA GACAAGCTT 1679

(2) INFORMATION FOR SEQ ID NO: 2:

(i) SEQUENCE CHARACTERISTICS:
(A) LENGTH: 912 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO: 2:

GATATCTTTA TTCCGCTATT CCTGTGCATC TGCCAGGAGC CCCTAGCGCA TAGATTTGTG 60

TGCAAATGAA ATGAGGATGT AGTCTGGGTG CCCAAGGGGG GTGCCTTGAG TGTGGTTGTC 120

TGTATGCCTC CCTGAGGGTA TTTCACTTTC TGCTCCCATC CGCCCCTATG AGCGAGTACC 180

TATGAGCACA GGATGTGCAC ATATTTGAGT CTTATTAGTG GTACACGCAG TTTTATCATC 240

TCCCCAGGTC TGTGTCTGTA TGAAATGTGC ATGGGTGTGT GTGTGCACGC GTGTGTTCCC 300

ACTCGGGGAA TGTGGGGAGA GGTGCATGGA GCCAAGATGG GTGGTAAATA GTATGTTTCT 360

GAAATTAAAG GACTAATGTG GAGGAAGGCG CCCCAGATGT ACGAAACCCT TTGCCCTTCA 420

TCTCATCCTC TCTGACTTGG GAAGAACCAG GATTTTGTTT TTAAGCCCTT GGGCATACAG 480

TTGTTCCATC CCGACATGAA CTCAGCCTCC CGTCTGACCG CCCCCTGGCC TTCCTTCTTC 540

CTCGATCTGT GGAACCCAGG GAATCTGCCT AGTGCTGTCT CCAAGCACCT TGGCCATGAT 600

GTAAACCCAG AGAAATTAGC ATCTCCATCT CCTTCCTTAT TCCCCACCCA AAAGTCATTT 660

CCTCTTAGTT CATTACCTGG GATTTTGATG TCTATGTTCC CTCCTCGTTA TTGATACACA 720

CACAGAGAGA GACAAACAAA AAAGGAACTT CTTGAAATTC CCCCAGAAGG TTTTGAGAGT 780

TGTTCTCAAT GTTGCGACAA GTCAGTTTCT AGTTTAAGTT TCCATCAGAA AGGAGTAGAG 840

TATATAAGTT CCAGTACCAG CAACAGCAGC AGAAGAAACA ACATCTGTTT CAGGGCCATT 900

GGACTCTCCG TC 912

(2) INFORMATION FOR SEQ ID NO: 3:

(i) SEQUENCE CHARACTERISTICS:
(A) LENGTH: 3641 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO: 3:

ATTGGACTCT CCGTCCTGCC CAGAGCAAGG TAAGCACTTC CCAAGCCCCT ACCTCCCTCC 60

CCTCCCTGTG GCCTGCAGCT GTCCAGGTGT AGAAACCGTT AGTGTGCTAC CCCAGCAGCT 120

GGCAGGAGGG AGTTGGTGGA TTCCTGGAAG CATCCCTGGT GAGTCATGTG CTGGAACATT 180

AGTGAAAACT TAGTACTCTA GGGACCGATG TACAGTGTCC ATTTTAAAAG CCACCTAATA 240

ATAACTGTAT AGCAAGATCT NNNNNNNNNN NNNNNNNNNN NNNNNNNNNN NNNNNNNNNN 300

NNNNNNNNNN NNNNNNNNNN NNNNNNNNNN NNNNNNNNNN NNNNNNNNNN NNNNNNNNNN 360

NNNNNNNNNN NNNNNNNNNN NNNNNNNNNN NNNNNNNNNN NNNNNNNNNN NNNNNNNNNN 420

NNNNNNNNNN NNNNNNNNNN NNNNNNNNNN NNNNNNNNNN NNNNNNNATC AACCAAAATG 480

ACAGCTGGGA TCCCCATTCT TGTGGATTAA CTAACTTTGG CCCCAGTTAA AAAATGAAAA 540

GCTATTATTG CTTCCTAAAG AGTTTTTAAA TCTGTGAGAA GGGGGAAAAA AAGGTTTTTT 600

TACTTGCCCA GGTAAAATTG TGTGCAACAA CAATGTCATT TTAACAAAGG GATTACTAAA 660

CCCCAGGTGA TGACCCACTT TTTCAAACAA GGAATTGCAA GATACTAGAT GGAATGTGGG 720

TAACCTCTTA GAGTTTAGTT TACTGGAATC TGAAATTGTA TGATTTCCGT ACATTCCTTC 780

ATGCTACTAA TAAGTCATGG AATCCCTCTG TGCTGGACTC TGGGGGTAAA GTGCAAAACA 840

AGATAGACAT GATCCATGGC ATAAGAGAGT TCACTCGGTG TGGCAGAGAA GACAAACAGT 900

AAAGAAGAGA CTGTATTGTT GCCATTGTAG TAAATGCTGT GGAAGGAGGG GAGCAAATAG 960

TGGGTCCTGA CTTCATCTAG GCACCATGAC ACTTCACGTG AATTATGTCA CTAACTCCTT 1020

ACCACAGTAG CATGCCCATT TTATAGATCA GGAGTGTGGG GCTTAAAGGG GCCAAGTGAC 1080

TCACCCAGAG TCACACAGTT CTAGAAGTCT GCCTGGCCCT CAAACTAGGG ATCTTTGCAT 1140

GTGCCACCCA TGCCATCTGT GACTATATCT TTTTATTCTT TAATAGTAAC TCCCTTTTCT 1200

AATTAAAGGT AACAAACAAA ACTTAAAAAA GAGATGCCCA CCCAAGCGTC ATTGGCATGC 1260

TGATGTTGGC ACCAGTGTTG GGAAGCCCTT AGCATACTCC AGGAAGTAGG AGTGTGTAAC 1320

GTGGGGTCCC TTTGTCCTTC ATGCAAGGGT TTCAAGAGTT TAGAAAACCT ATGAAATTGC 1380

ACACACAAAA ATGTGTTTTA ATCATCAAGA CTCTCAGACT TACCATGCTG AGAAATGTGG 1440

GCTGAGGGAT AGTGCTCCCT AGTATCAGCT GATGGGCCAG AGAGCCAAAG GAGGAGACCC 1500

ACCACCATCA CCTCTCCTGG ACAGTGCTCT GTGGTTTCAA ATGTAGGTGA TACTAAAATG 1560

GGAGTTGTTT CTTAGAACCA TGGCCGGGGT TCCCTTGACC CTGAAGTGCA GTTCACCTGA 1620

GATTGTCAAA TGCATCTGAG GCATGCAGAG GAAGTGCTGG GCACACAGCT AGTGGGAATA 1680

CCTCAGCGTA AGTGGCCAGG AGATGCCAGG AATCTCCACT ATTTCCCTTC CAGTGTGCCA 1740

GCCTCTGGGT TTTACAGGCG CATGTAATTG CAGTACCTCT GTGCACATTT CCCTACTGCC 1800

TAGAATGACT TTCTTGACTA TCCATGATAT ATAAAACACA GATACCAAAT TGTTCCCTTA 1860

CCTCTTCCTC TAGGTTCAAG TTAATATGTT ATTGGTTGCC TTCTATAATA TGTTATCTCA 1920

TTGCCTTTCC CAACAAGCCT TTGAGATAAG TATTAGGTCC ATTTTATAGA CAAAGAGACT 1980

GAGGCTCAGC GAGTAACTTG GCCAACAAGT TGCTCCCACT GCTCAACAGC AAATGAGCGG 2040

TGGGACCAAA ATTCAAGCCT GTGTCTGTTT AACTTCAAGC CTGTGAATGT ACTAACCGGT 2100

GCCCTGTGCC AGCTAGTACT TTGCTACAGT CATAACCTAG ACTGAAGTGA TAGCCATGCC 2160

CCTAAAACTC CATGCTGTGG TGACAGCACT GAGCAGTGTC CAAGAAGGCT TGACTTCTAG 2220

GCCTGTCTCT GCCACTCACA AACTTTATAA GGGAATAAAG TACATAGCAA GGTCCGCCTA 2280

GAGTTAGTAG CAGTTCTGAC AAAGCTGTAA TTTGTCAATA TTCCGTCACC CAACCCAGGA 2340

ATGCTCATTT TTAAGGTATT TGACTGAAAC AGTTGAGCAT TGCCCTTCAT ATAGTTTAAA 2400

ACAGTGGTTT CAGAACCACT TTCCTCCAGA CCATGGGTGC TCTGCAAGGT GAATGGAGTT 2460

GTTTCAGAAT GTTTCAATAA TCATCCCTAC CTCATTCGTA AGTGGCATGT AATTTTTGCA 2520

ATCGGAAGAT TTTCATAAAC CCTGGATACT AACCTAGACT GGTTTCTATA TCAGATGGTG 2580

GCTTATTTAA CATAAAATTA TGCATTTTAC TATTTCATGG TGGATATATC AATATGTTGT 2640

GGTCTTTTCC CAATGAACAC TTTGATTTTC AGGGGTTCTG GACCCTGAAC ATGGGTTAAA 2700

CCAGTGGTTC TCAAGGTGTG GTCTTAGCGC CAGCAGCATC TGCTTCCCCT GGAAACTTTC 2760

TAGAAATGCA TATTCTCAGG CCCTCATGCC TGCTGAATCA GACACTCTGG GGGTGGGACT 2820

CAGCCGTCTG TTGTAGCAGT GCTTCCAGGT TATCCTGACA GTCACTCAAA TTTAGAACCA 2880

CTAGGTTCTC TATATGGGAG AGAGTAGTCT TTGAACTTGG AAAACAAGAG AAGCTAAACC 2940

CCTACAGCAA GGGCTGGTGA CCAGGTCGTT GCCAGAACCT GAAAGTTCGC CTCTGTATTA 3000

CCGTTCCTGT CCCTAACCCA AGTCCTTCAG TTCTGGGTGC TCCAGCACAC ACTGCTTTGT 3060

GCTGCAGTGA TACAAATGTA TGGCTCATCT CCCCAGCTGG CGGGGAGGCA TTTAACACAC 3120

TGACTTAATA AATATTTATT GAGTAAAAGT ATTTGCTCCT AGGAAGCGGG ATCCAGGTAA 3180

GCCCTTTTTT TCTCTCTCAA CTGCTTCTAG CCCAGTGCTC TTTATGTAGT AAGCACTAAA 3240

TAAACAACTG CTAGATGTTG ATCCAGAAAG TCACATTCCT TCTCTAAGCT TTAAGTTTCT 3300

CATCTTAAAA ATAAGAGGAT TGTATCAGAT GGCTTGCCTT AGGTCTCTTT CAGCTCCAGA 3360

GCCCCAAATA CCCTATGGTT CTCTATTTAG AGATGTTCTT CCCCACAGAC TGCCATAGAA 3420

CTCCTGTAAT TTACTTAGTA TTTGCTTGAC AGTATGGAGA AGAAAGGGGA GAATCAAGAT 3480

TTTATTTAAA AAAAAAGTAG CTAGAATGTG TATATGGTTC ACAAAGGTAA CAAGAATTAT 3540

TGACATTCTT TCTTCTCTTT TTTCTTCCTC TTCCTTCTCT TTTCCTCCTT CTCTTCCCCC 3600

TGCTTCTCTC CCTTCTTATA GATGTGTCAT CCAGCAGTTG G 3641